# FASCICULE DE BREVET EUROPEEN

(11) **EP 0 532 440 B1**
(45) Date de publication et mention de la délivrance du brevet: **08.11.1995**
(21) Numéro de dépôt: 92420311.0
(22) Date de dépôt: 11.09.1992
(51) Int. Cl.: A61F 2/42

(54) **Prothèse totale du poignet**
Handgelenkprothese
Total wrist prosthesis

(30) Priorité: 12.09.1991 FR 9111465
(43) Date de publication de la demande: 17.03.1993
(73) Titulaire: TORNIER SA, F-38330 Saint Ismier (FR)
(72) Inventeur: Judet, Thierry, F-92410 Ville d'Avray (FR)
(74) Mandataire: Monnier, Guy

(56) Documents cités:
- DE-A- 2 309 432
- US-A- 4 021 864
- US-A- 4 040 130
- US-A- 4 259 752
- US-A- 4 307 473

## Description

La présente invention concerne une prothèse totale du poignet qui comporte plus particulièrement des moyens permettant la reproduction mécanique de la cinématique d'un poignet sain.

On connaît des prothèses de ce genre qui comprennent plus généralement un bloc de matériau élastique réalisé par exemple dans une matière dénommée "silastic". Ces prothèses ne s'adaptent pas correctement à l'environnement osseux, tandis que leur conception reste une approche rudimentaire de la cinématique du poignet.

En outre, d'autres prothèses sont réalisées en métal assimilant le poignet à une articulation sphérique ne permettant pas une cinématique normale et limitant de ce fait la fonction du poignet.

On connaît aussi, d'après le brevet américain US 4 307 473 des prothèses qui comportent trois éléments présentant chacun des surfaces de glissement distinctes pour les mouvements de flexion-extension et de déviatior radio-cubitales. Ces surfaces de glissement comportent certains inconvénients car elles sont d'une part limitées dans leurs degrés de liberté et d'autre part seulement centrées suivant un point unique, ce qui empêche la reproduction de la cinématique normale d'un poignet sain.

C'est à ces inconvénients qu'entend plus spécialement remédier la présente invention.

L'invention a pour but de réaliser une prothèse totale du poignet comportant trois éléments distincts. Ces éléments permettent à l'ensemble des structures osseuse, ligamentaire ou musculaire restant en place de guider le mouvement du poignet sans soumettre l'implant à des contraintes risquant d'handicaper le patient.

En outre les trois éléments offrent des centres de rotation distincts pour les deux mouvements principaux du poignet tandis que l'élément intermédiaire ou second élément est totalement libre en rotation dans deux plans perpendiculaires permettant le déplacement radio-cubital et dorso-palmaire du centre articulaire médio-carpien au cours du mouvement.

Le second élément présente une forme de tonneau conique coopérant avec une surface extérieure concave suivant ses deux axes principaux, ladite surface étant usinée dans une plaque recouvrant le plateau du premier élément, permettant de définir au moins deux centres de rotation différents du second élément, dont l'un se trouve dans son plan sagittal tandis que l'autre est prévu dans son plan frontal de manière à reproduire respectivement une rotation en flexion-extension et une rotation en déviation radio-cubitale.

En outre le second élément comporte dans son plan frontal une courbure en arc de cercle d'un rayon supérieur à celui prévu dans son plan sagittal.

De plus le second élément comporte un évidement qui est décalé d'une part dans le plan frontal soit à droite, soit à gauche respectivement pour une main droite ou une main gauche et d'autre part dans le plan sagittal en direction du haut de façon à récupérer le décalage angulaire existant entre le radius, les os des carpes et les métacarpiens.

Le dessin annexé, donné à titre d'exemple, permettra de mieux comprendre l'invention, les caractéristiques qu'elle présente et les avantages qu'elle est susceptible de procurer :
Fig. 1 est une vue en perspective éclatée illustrant les différents éléments composant la prothèse totale de poignet suivant l'invention.
Fig. 2 est une vue de dessus avec arrachements montrant la résection pratiquée sur le radius et sur les première et deuxième rangées du carpe d'une main gauche pour la mise en place de la prothèse de fig. 1.
Fig. 3 est une coupe suivant III-III (fig. 2) représentant le décalage angulaire dans le plan sagittal entre la partie fixe et les parties mobiles de la prothèse conforme à l'invention.

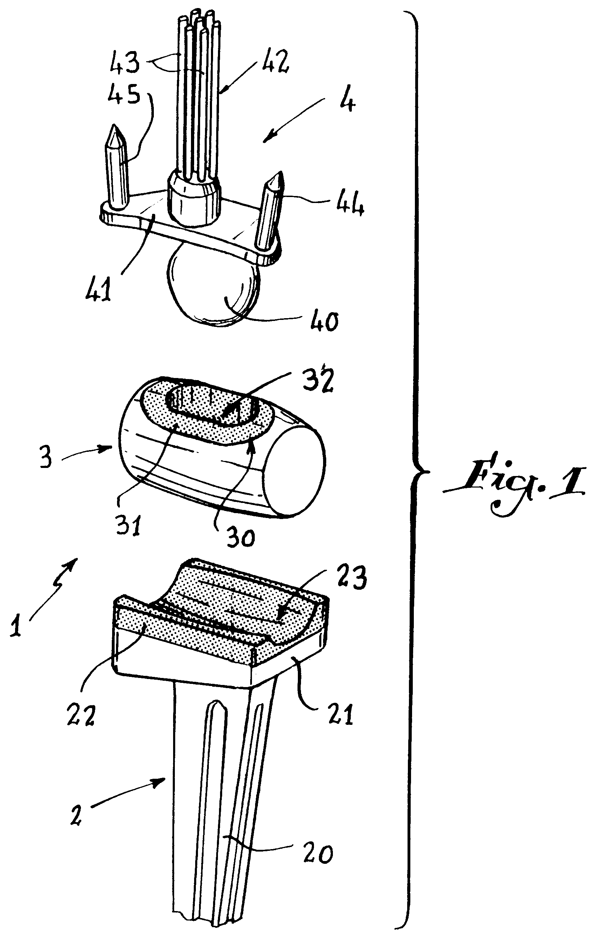

On a représenté en fig. 1 une prothèse totale du poignet 1 comportant trois éléments 2, 3 et 4 s'articulant les uns dans les autres en vue de reproduire parfaitement les deux articulations du poignet.

Le premier élément 2 comprend d'une part une tige d'ancrage 20 de forme allongée et adaptée à la morphologie interne du radius et d'autre part un plateau 21 solidaire de la tige qui vient en appui contre la coupe osseuse de la surface articulaire radiale, comme on le verra mieux plus loin.

L'élément 2 est réalisé en une matière métallique de manière que la surface de la tige d'ancrage 20 comporte des dessins permettant la mise en place soit avec ciment, soit sans ciment à l'intérieur de la diaphyse radiale.

Le plateau 21 est recouvert d'une plaque 22 qui peut être soit intégrée, soit rapportée à l'élément 2. La plaque 22 est fabriquée en une matière plastique telle que du polyéthylène ou analogue destinée à réaliser la surface de glissement radio-carpienne concave 23.

A cet effet, la surface 23 présente un profil extérieur concave suivant ses deux axes principaux.

Le second élément 3 présente une forme de tonneau conique afin de coopérer parfaitement avec la surface 23 de la plaque 22. La configuration du tonneau montre dans son plan frontal une courbure en arc de cercle d'un rayon supérieur à celui prévu dans son plan sagittal.

Ainsi au niveau articulaire radio-carpien le second élément 3 comporte un premier centre de rotation en déviation radio-cubitale qui est situé dans son plan frontal le plus éloigné et un second centre de rotation en flexion-extension qui se trouve dans son plan sagittal c'est-à-dire à l'intérieur dudit élément 3.

Du fait de l'obliquité de l'interligne radio-carpien, l'élément 3 comporte une génératrice oblique pour rétablir l'anatomie.

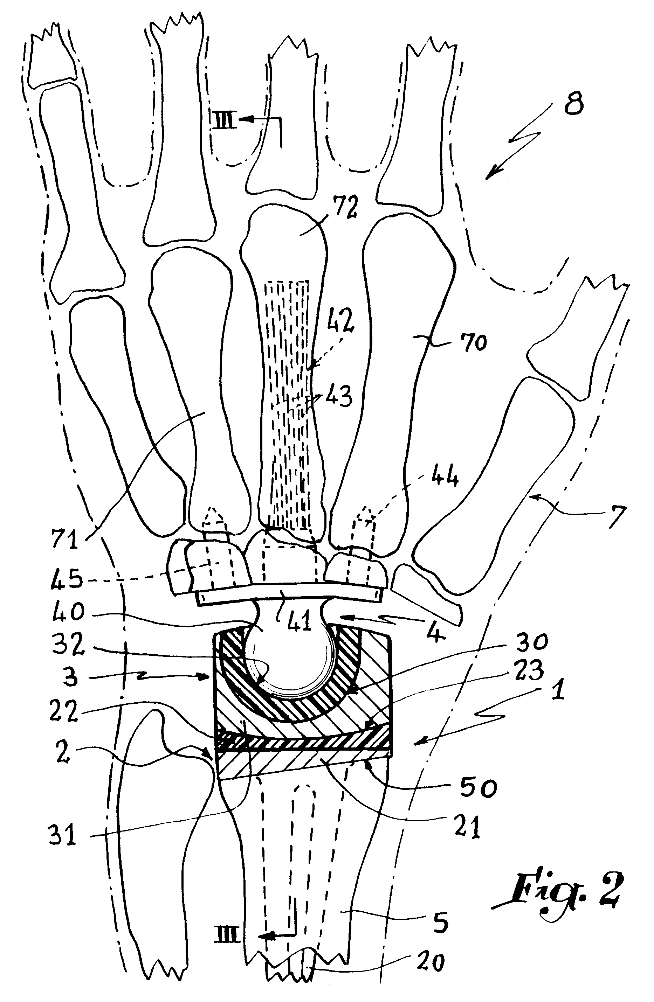

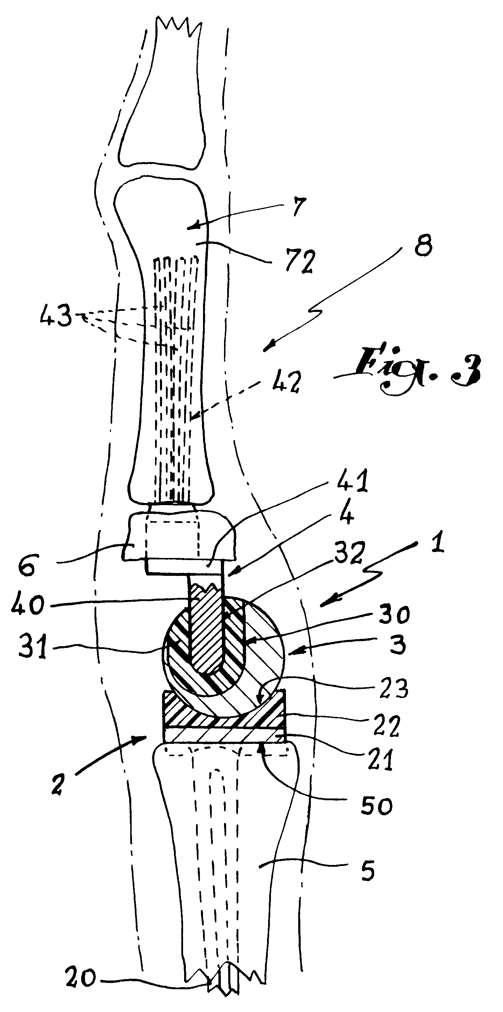

Un évidement oblong 30 est ménagé dans l'élément 3 de manière à être décalé angulairement dans le plan frontal soit à droite, soit à gauche suivant l'anatomie de l'articulation du poignet et de la main (fig. 2). On remarque de plus que l'évidemment 30 est déplacé en direction du haut dans le plan sagittal de la main (direction dorsale) de façon à récupérer le décalage angulaire existant entre le radius 5, les os des carpes 6 et les métacarpiens 7 (fig. 3).

En outre, le déplacement en direction du haut de l'évidement 30 donne à l'élément 3 une fonction identique à celle de l'os semi-lunaire, dans lequel le centre de rotation de la tête du grand os est décalé dorsalement par rapport au centre de rotation radio-lunaire.

A l'intérieur de l'évidement 30 est introduit à force un insert 31 réalisé en une matière telle que le polyéthylène ou analogue. Cet insert comporte en son milieu un logement oblong 32 permettant de réaliser la surface de glissement médio-carpienne.

Cette surface de glissement est conçue pour recevoir un troisième élément 4 articulé de manière à constituer une rotation se trouvant dans le plan horizontal du poignet c'est-à-dire de gauche à droite et inversement dans le plan frontal de la main 8 (fig. 2). Le centre de cette rotation prévu dans le plan horizontal correspond au centre de l'évidement 30 de l'élément 3 qui se trouve décalé dorsalement par rapport aux centres de rotation articulaires radio-carpien décrits précédemment. L'élément 4 comprend essentiellement un disque épais 40 métallique qui vient s'introduire dans le logement oblong 32 du second élément 3 en vue de réaliser la rotation comprise dans le plan horizontal sus-mentionné du poignet. Le disque 40 est solidaire d'une plaque 41 légèrement courbe et qui est décalée en hauteur par rapport à l'axe de pivotement dudit disque à l'intérieur du logement oblong 32 (fig. 3).

La plaque 41 comporte dans sa partie supérieure et en son milieu une tige 42 composée d'un faisceau de brins métalliques souples 43, tandis que de part et d'autre de celle-ci sont fixés deux picots rigides 44 et 45. Ces picots peuvent être éventuellement remplacés par des vis passant ou non au travers de chevilles à expansion.

En fig. 2 et 3, on a représenté la prothèse totale du poignet 1 d'une main gauche 8. On remarque qu'il faut pour la mise en place de la prothèse 1, réséquer l'extrémité du radius 5 pour que le plateau 21 solidaire de la tige 20 du premier élément 2 vienne en appui contre la coupe 50 du radius 5. L'inclinaison du plateau 21 dépend bien évidemment de la mise en place de la prothèse 1 dans une main gauche 8 ou dans une main droite, comme illustré sur les figures. Ensuite, le chirurgien procède à l'élimination de la rangée supérieure ou anti-brachiale du carpe et d'une partie de la rangée inférieure ou carpienne 6. L'élimination de la rangée supérieure du carpe permet la mise en place du second élément 3 pour réaliser l'articulation radio-carpienne du poignet. A l'intérieur du logement oblong 32 prévu dans l'élément 3 est introduit le disque épais 40 du troisième élément 4 de manière que les picots ou vis 44 et 45 viennent s'ancrer dans la partie restante de la rangée inférieure du carpe 6 et dans les métacarpiens 70 et 71 se trouvant de part et d'autre du majeur 72. A l'intérieur de la diaphyse métacarpienne du majeur 72 est introduite la tige 42 qui comporte le faisceau de brins métalliques 43. Ce dernier peut être adapté suivant la longueur et la largeur de la diaphyse en coupant un certain nombre de brins 43 avec une simple pince. La mise en place du troisième élément 4 permet de réaliser l'articulation médio-carpienne du poignet. En fig. 3, on remarque que l'axe de mouvement du troisième élément 4 composant l'articulation médio-carpienne est décalé dorsalement par rapport à l'axe de mouvement du second élément 3. Ce décalage autorise ainsi l'élément 4 à effectuer un mouvement de flexion-extension proche de l'anatomie, tel que l'engendrent les os (scapholïdes de la rangée supérieure du carpe semi-lunaire), d'un poignet sain. On note par ailleurs que la prothèse totale du poignet 1 selon la présente invention permet, grâce à ses deux surfaces de glissment, une grande liberté de mouvements.

On remarquera que l'élément 3 n'est lié à aucune structure osseuse ou ligamentaire et dispose d'au moins deux degrés de liberté en rotation par rapport à l'élément radial 2. De plus ceci combiné au fait que les centres de rotation radio-carpien et médio-carpien sont distincts permet au centre de rotation médio-carpien de se positionner ou de se mouvoir de lui-même dans la limite de ses degrés de liberté, en fonction des contraintes imposées par les structures anatomiques restant en place.

## Revendications

1. Prothèse totale du poignet du genre comprenant un premier élément (2) pourvu d'un plateau (21) et d'une tige d'ancrage adaptée à la morphologie interne du radius, un second élément (3) coopérant avec le plateau (21) pour son pivotement par rapport au premier élément et un troisième élément (4) libre en rotation dans un plan frontal par rapport au second élément, caractérisée en ce que le second élément (3) présente une forme de tonneau conique qui coopère avec une surface exterieure (23) concave suivant ses deux axes principaux, ladite surface étant usinée dans une plaque (22) recouvrant le plateau (21) du premier élément (2), permettant de définir au moins deux centres de rotation différents du second élément (3), dont l'un se trouve dans son plan sagittal tandis que l'autre est prévu dans son plan frontal de manière à reproduire respectivement une rotation en flexion-extension et une rotation en déviation radiocubitale.

2. Prothèse suivant la revendication 1, caractérisée en ce que le second élément (3) comporte dans son plan frontal une courbure en arc de cercle d'un rayon supérieur à celui prévu dans son plan sagittal.

3. Prothèse suivant la revendication 1, caractérisée en ce que le second élément (3) comporte un évidement (30) qui est décalé d'une part dans le plan frontal soit à droite, soit à gauche respectivement pour une main droite ou une main gauche (8) et d'autre part dans le plan sagittal en direction du haut de façon à récupérer le décalage angulaire existant entre le radius (5), les os des carpes (6) et les métacarpiens (7).

4. Prothèse suivant la revendication 3, caractérisée en ce que l'évidement (30) reçoit un insert (31) réalisé en une matière plastique.

5. Prothèse suivant la revendication 3, caractérisée en ce que l'évidement (30) est destiné à recevoir le troisième élément (4) afin qu'il soit libre en rotation dans le plan sagittal par rapport au second élément (3).

6. Prothèse suivant la revendication 1, caractérisée en ce que le troisième élément (4) comprend un disque épais (40) solidaire d'une plaque (41) décalée en hauteur par rapport à l'axe de pivotement dudit disque et sur laquelle sont fixés une tige (42) constituée d'un faisceau de brins métalliques souples (43) et au moins deux picots (44, 45) placés de part et d'autre de ladite tige.

7. Prothèse suivant la revendication 1, caractérisée en ce que la plaque (22) recouvrant le plateau (21) du premier élément (2) est réalisée en une matière bio-compatible.

8. Prothèse suivant la revendication 1, caractérisée en ce qu'elle est entièrement maintenue dans ses déplacements par l'ensemble des structures osseuse, ligamentaire et musculaire restant en place après la résection.

## Claims

1. Total wrist prosthesis of the type comprising a first element (2) provided with a plateau (21) and an anchoring rod adapted to the internal morphology of the radius, a second element (3) cooperating with the plateau (21) for its pivoting with respect to the first element, and a third element (4) free in terms of rotation in a frontal plane with respect to the second element, characterized in that the second element (3) has a conical barrel shape which cooperates with an outer surface (23) concave in the direction of its two main axes, the said surface being machined in a plate (22) covering the plateau (21) of the first element (2), making it possible to define at least two different centres of rotation of the second element (3), of which one is situated in its sagittal plane, while the other is provided in its frontal plane, in such a way as to reproduce, respectively, a flexion/extension rotation and a cubitoradial deflection rotation.

2. Prosthesis according to Claim 1, characterized in that the second element (3) comprises in its frontal plane a curvature in the form of an arc of a circle with a radius greater than that provided in its sagittal plane.

3. Prosthesis according to Claim 1, characterized in that the second element (3) comprises a recess (30) which is offset, in the first place, in the frontal plane, either to the right or to the left for a right hand or a left hand (8), respectively, and, in the second place, in the sagittal plane in an upward direction in such a way as to recover the angular offset existing between the radius (5), the carpal bones (6) and the metacarpals (7).

4. Prosthesis according to Claim 3, characterized in that the recess (30) receives an insert (31) made of a plastic material.

5. Prosthesis according to Claim 3, characterized in that the recess (30) is intended to receive the third element (4) so that it is free in terms of rotation in the sagittal plane with respect to the second element (3).

6. Prosthesis according to Claim 1, characterized in that the third element (4) comprises a thick disc (40) integral with a plate (41) which is offset upwardly with respect to the axis of pivoting of the said disc and on which are fixed a rod (42), consisting of a bundle of flexible metal strands (43), and at least two spikes (44, 45) placed on either side of the said rod.

7. Prosthesis according to Claim 1, characterized in that the plate (22) covering the plateau (21) of the first element (2) is made of a biocompatible material.

8. Prosthesis according to Claim 1, characterized in that it is completely supported in its displacements by the combination of the bone, ligament and muscle structures which remain in place after the resection.

## Patentansprüche

1. Totalprothese des Handgelenks von der Art, die ein erstes Element (2) aufweist, das mit einer Hochfläche (21) und einem Ankerschaft versehen ist, der der inneren Morphologie des Radius angepaßt ist, die ein zweites Element (3) aufweist, das mit der Hochfläche (21) zusammenwirkt, um gegenüber dem ersten Element zu schwenken, und die ein drittes Element (4) aufweist, das gegenüber dem zweiten Element in einer Frontalebene frei rotieren kann,
dadurch **gekennzeichnet,**
daß das zweite Element (3) eine konische Tonnenform aufweist, die mit einer gemäß ihrer beiden Hauptachsen konkaven Außenoberfläche (23) zusammenwirkt, die in einer die Hochfläche (21) des ersten Elements (2) bedeckenden Platte (22) ausgebildet ist, wodurch mindestens zwei unterschiedliche Rotationszentren des zweiten Elements (3) definiert werden können, von denen sich eines in dessen Sagittalebene befindet, während das andere in dessen Frontebene derart vorgesehen ist, daß jeweils entsprechend eine Beuge-Steck-Rotation und eine radiocubitale Deviationsrotation reproduziert werden können.

2. Prothese gemäß Anspruch 1,
dadurch **gekennzeichnet,**
daß das zweite Element (3) in seiner Frontalebene eine Kreisbogenkrümmung mit größerem Radius als dem aufweist, der in seiner Sagittalebene vorgesehen ist.

3. Prothese gemäß Anspruch 1,
dadurch **gekennzeichnet,**
daß das zweite Element (3) eine Aussparung (30) aufweist, die einerseits in der Frontalebene jeweils entsprechend für eine rechte oder eine linke Hand (8) entweder nach rechts oder nach links versetzt ist und die andererseits in der Sagittalebene derart nach oben hin versetzt ist, daß die zwischen dem Radius (5), den Carpal- (6) und den Metacarpalknochen (7) bestehende Winkelabweichung erhalten wird.

4. Prothese gemäß Anspruch 3,
dadurch **gekennzeichnet,**
daß die Aussparung (30) einen aus einem Kunststoff hergestellten Einsatz (31) aufnimmt.

5. Prothese gemäß Anspruch 3,
dadurch **gekennzeichnet,**
daß der Einsatz (30) zur Aufnahme eines dritten Elements (4) bestimmt ist, damit dieses in der Sagittalebene gegenüber dem zweiten Element (3) frei rotieren kann.

6. Prothese gemäß Anspruch 1,
dadurch **gekennzeichnet,**
daß das dritte Element (4) eine mit einer Platte (41) verbundene dicke Scheibe (40) aufweist, die im Verhältnis zur Schwenkachse der Scheibe nach oben hin versetzt ist und auf der ein aus einem Bündel von biegsamen metallischen Stiften (43) gebildeter Stab (42) und mindestens zwei Pfähle (44,45), die beidseits des Schaftes angeordnet sind, befestigt sind.

7. Prothese gemäß Anspruch 1,
dadurch **gekennzeichnet,**
daß die die Hochfläche (21) des ersten Elements (2) bedeckende Platte (22) aus einem biokompatiblen Material hergestellt ist.

8. Prothese nach Anspruch 1,
dadurch **gekennzeichnet,**
daß sie während ihrer Verschiebungen vollständig von den gesamten Knochen-, Band- und Muskelstrukturen, die nach der Resektion an ihrer Position bleiben, gehalten wird.
